# EUROPÄISCHE PATENTSCHRIFT

(11) **EP 0 839 181 B1**
(45) Veröffentlichungstag und Bekanntmachung des Hinweises auf die Patenterteilung: **21.11.2001**
(21) Anmeldenummer: 97917973.6
(22) Anmeldetag: 07.05.1997
(51) Int. Cl.: C12M 1/22, C12M 1/36, C12Q 1/02

(54) **GERÄT ZUR BESTIMMUNG DER ZAHL VON MIKROORGANISMEN IN LUFT SOWIE EIN VERFAHREN ZUM BETRIEB DIESES GERÄTES**
APPARATUS FOR DETERMINING THE NUMBER OF MICROORGANISMS IN THE AIR AND A METHOD OF OPERATING SAID APPARATUS
APPAREIL PERMETTANT DE DETERMINER LE NOMBRE DE MICRO-ORGANISMES PRESENTS DANS L'AIR ET PROCEDE DE FONCTIONNEMENT DUDIT APPAREIL

(30) Priorität: 07.05.1996 CH 116396
(43) Veröffentlichungstag der Anmeldung: 06.05.1998
(73) Patentinhaber: Pitzurra, Ovidio, 9450 Altstätten SG (CH)
(72) Erfinder: Pitzurra, Ovidio, 9450 Altstätten SG (CH)
(74) Vertreter: Kulhavy, Sava V.
(86) Internationale Anmeldenummer: CH9700176
(87) Internationale Veröffentlichungsnummer: WO9742304

(56) Entgegenhaltungen:
- DE-A- 3 628 155
- DE-C- 4 300 231
- US-A- 4 468 914

## Beschreibung

Die vorliegende Erfindung betrifft ein Gerät zur Bestimmung der Zahl von Mikroorganismen in Luft gemäss dem einleitenden Teil des Patenanspruchs 1 sowie ein Verfahren zum Betrieb dieses Gerätes.

Ein Gerät dieser Gattung ist beispielsweise in US-PS 4,468,914 offenbart. Bei diesem vorbekannten Gerät können sich Petri-Schalen eine nach der anderen, in Abständen voneinander und kontinuierlich entlang einer Bahn bewegen. Die Petri-Schalen bewegen sich gegen ein Paar in einem Abstand voneinander angeordneten Versorgungsdüsen hin. An einer bestimmten Stelle der Bewegungsbahn, und zwar noch vor den Düsen, gelangen die Deckel der Petri-Schalen mit einer Rampe in Eingriff, welche den Deckel der jeweiligen Petri-Schale anhebt, damit in der Petri-Schale ein Raum für die Aufnahme der Düsen entsteht. Durch diese Düsen gelangt ein Wachstumsmedium für Bakterien in die Petri-Schalen. Hiernach wird der Deckel auf die Petri-Schale wieder aufgesetzt. Dieses vorbekannte Gerät dient somit bloss zum Füllen der Petri-Schalen mit einem Wachstumsmedium und es hat mit der Bestimmung der Zahl von Mikroorganismen in der Luft somit nichts zu tun.

Zur Feststellung der Anzahl von Mikroorganismen, insbesondere von Keimen wie Pilzen, Bakterien usw. in Luft ist es allgemeine bekannt, Mikroorganismen, welche in der Luft schweben, während einer vorbestimmten Zeitspanne auf einem Kulturboden ablagern zu lassen. Dann wird dieser Kulturboden abgedeckt und Bedingungen ausgesetzt, welche ein Vermehren der abgelagerten Mikroorganismen begünstigen. Nach Ablauf einer vorgegebenen Zeitspanne werden Herde bzw. Bezirke auf dem Kulturboden gezählt, welche aus dem Wachstum der abgelagerten Mikroorganismen resultieren.

Zur Durchführung dieses Verfahrens wird eine sogenannte Petri-Schale verwendet, welche einen Unterteil mit einem Nährboden sowie einen Deckel aufweist. Man nimmt den Deckel vom Unterteil weg und lässt die Petri-Schale während einer gewünschten Zeitspanne offen. Dann macht man die Petri-Schale von Hand wieder zu. Die Genauigkeit dieser Art von Messung der Anzahl von Keimen in Luft hängt weitgehend von Sorgfältigkeit jener Person ab, welche diese Messungen manuell durchführt. Ausserdem erfordert dieses vorbekannte Verfahren, dass die solche Messungen durchführende Person während des gesamten Messverfahrens anwesend ist. Dies ist insbesondere dann ungünstig, wenn solche Messungen ausserhalb der gewöhnlichen Arbeitszeit, an schwer zugänglichen Stelle usw. durchgeführt werden müssen.

In DE-A 36 28 155 ist eine Vorrichtung zum Exponieren von Kulturschalen offenbart. Die in dieser Vorrichtung verwendeten Kulturschalen weisen lediglich den Unterteil und somit keinen Deckel auf. Ausserhalb der Expositionszeit befindet sich die Kulturschale in einem Gehäuse, welches unter anderem verhindert, dass Mikroorganismen ausserhalb der Expositionszeit zum sich in der Schale befindlichen Wachstumsmedium gelangen. Im Gehäuse ist ein aus- und einfahrbares Gestell angeordnet, auf dem sich die Kulturschale befindet. Um das Wachstumsmedium in der Kulturschale den Mikroorganismen auszusetzen, transportiert das Gestell die Kulturschale aus dem Gehäuse heraus, wo die Schale während einer gewünschten Zeitspanne (Expositionszeit) dem Einfluss der Mikroorganismen ausgesetzt wird. Die Zeitspanne, während welcher der Nährboden in der Schale die Mikroorganismen empfangen kann, ist durch jene Zeitspanne bestimmt, während welcher die Schale aus dem Gehäuse ausgefahren ist.

Zur Bestimmung der Menge der eingefangenen Mikroorganismen muss die offene Kulturschale aus dem Gehäuse herausgenommen und zur weiteren Behandlung transportiert werden. Während diesem Transport können weitere Mikroorganismen in die Kulturschale gelangen, wodurch die Messresultate in Bezug auf den ursprünglichen Aufstellungsort des Gehäuses verfälscht werden können.

Die Aufgabe der vorliegenden Erfindung ist, die genannten sowie noch weitere Nachteile des Standes der Technik zu beheben.

Diese Aufgabe wird beim Gerät der eingangs genannten Gattung erfindungsgemäss so gelöst, wie dies im kennzeichnenden Teil des Patentanspruchs 1 definiert ist.

Das erfindungsgemässe Verfahren ist im Patentanspruch 18 definiert.

Nachstehend werden Ausführungsformen der vorliegenden Erfindung anhand der beiliegenden Zeichnungen näher erläutert. Es zeigt:
Fig. 1 in einer Seitenansicht eine erste Ausführung des vorliegenden Gerätes,
Fig. 2 das Gerät aus Fig. 1 in einer Draufsicht,
Fig. 3 in einer Seitenansicht eine zweite Ausführung des vorliegenden Gerätes,
Fig. 4 das Gerät aus Fig. 3 in einer Draufsicht,
Fig. 5 teilweise in einem vertikalen Schnitt den mechanischen Teil des vorliegenden Gerätes,

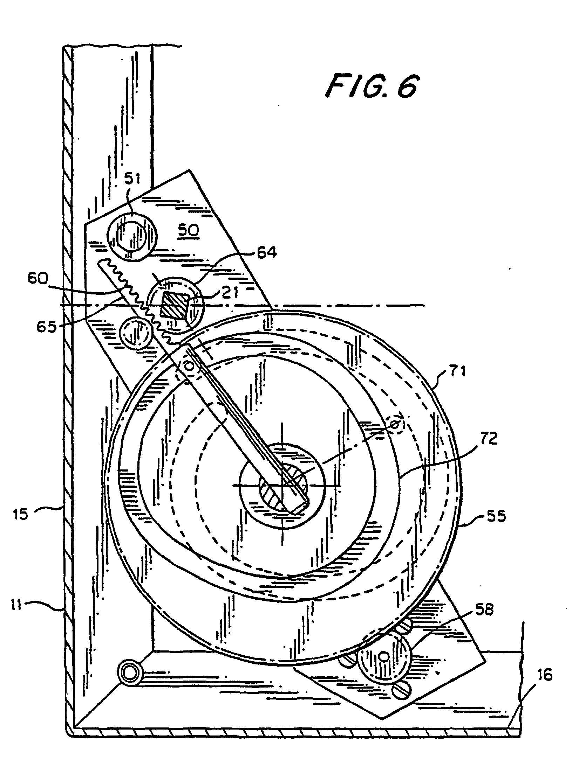

Fig. 6 in einer Draufsicht den unteren Abschnitt des mechanischen Teiles aus Fig. 5,

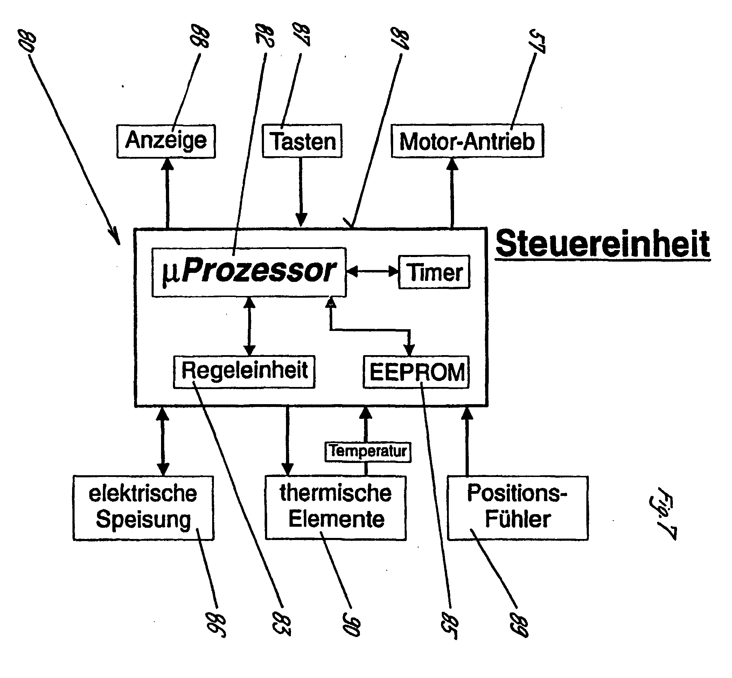

Fig. 7 in einem Blockschaltbild den elektrischen Teil des vorliegenden Gerätes,
Fig. 8 eine Korrelationstabelle und
Fig. 9 bis 14 eine zweite Betätigungseinrichtung.

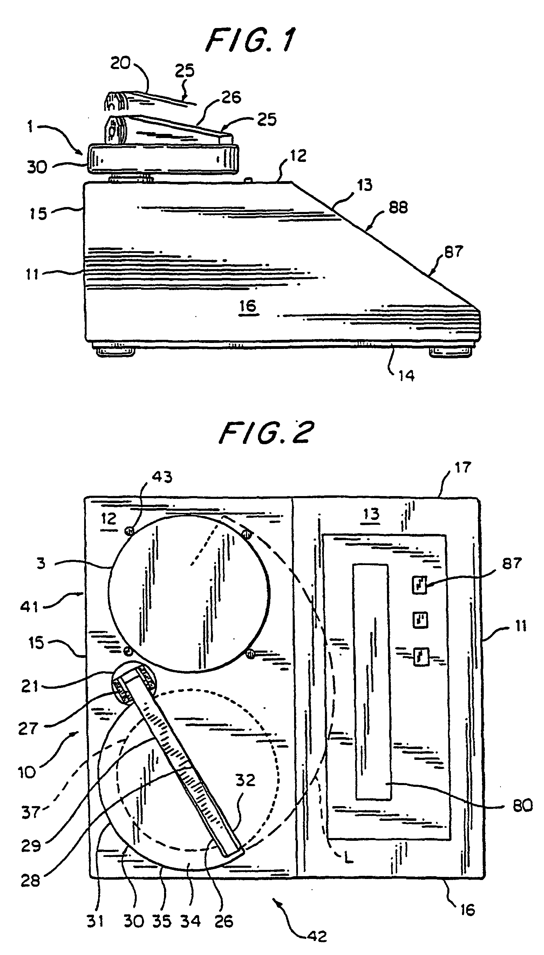

Fig. 1 zeigt in einer Seitenansicht eine erste Ausführung des vorliegenden Gerätes, während Fig. 2 das Gerät aus Fig. 1 in einer Draufsicht zeigt. Dieses Gerät dient zur Bestimmung der Anzahl von Keimen in Luft und es umfasst eine Vorrichtung 1 zur Ablagerung von Keimen sowie eine Einrichtung 10 zur Betätigung dieser Ablagerungsvorrichtung 1.

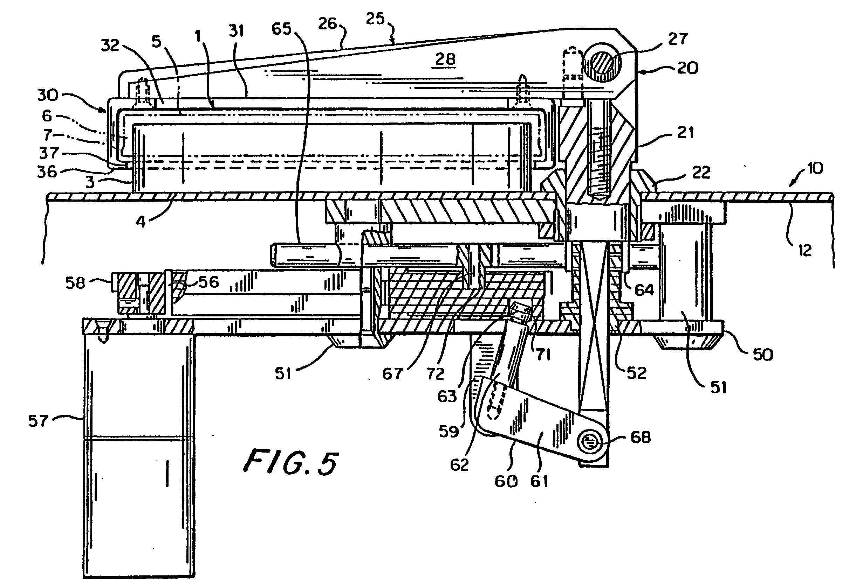

Die Ablagerungsvorrichtung 1 ist im vorliegenden Fall als eine an sich bekannte Petrischale ausgeführt. Die Petrischale 1 weist einen Unterteil 3 (Fig. 5) sowie einen Deckel 5 auf. Die Oberseite des Bodens des Schalenunterteiles 3 ist mit einem Kultur- bzw. Nährboden 4 bedeckt, auf welchem Mikroorganismen sedimantieren können, wenn die Petrischale offen steht. Auf dem Nährboden 4 können sich dann die sedimentierten Mikroorganismen vermehren, wenn die Petrischale 1 geschlossen ist. Der Kulturboden 4 liegt vorteilhaft in einer horizontalen Ebene.

Die Ablagerungsvorrichtung 1 ist ferner so ausgeführt, dass während der Inkubationszeit keine Keime bzw. Partikeln mehr in diese Vorrichtung 1 gelangen können. Zu diesem Zweck ist der Deckel 5 der Petrischale 1 so ausbildet, dass auch seine herabhängende Randpartie 6 verhindert, dass Keime während der Inkubationszeit auf den Unterteil 3 gelangen. Dabei kann der Deckel 5 auf dem oberen Rand des Schalenunterteiles 3 entweder dicht oder unter Belassung eines Spaltes zwischen dem Deckel 5 und dem Unterteil 3 aufliegen. Zur Bildung dieses Spaltes ist die Unterseite des Schalendeckels 5 mit Vorsprüngen in einer an sich bekannten Weise versehen.

Die Betätigungseinrichtung 10 umfasst ein Gehäuse 11, welches eine horizontal verlaufende Oberwand 12, eine Display- bzw. Anzeigewand 13, einen Boden 14, eine Rückwand 15 sowie Seitenwände 16 und 17 aufweist. Die Anzeigewand 13 verläuft vom Gehäuseboden 14 schräg rückwärts, sodass die Länge bzw. Tiefe der horizontal verlaufenden Oberwand 12 kleiner ist als die Länge bzw. Tiefe des Bodens 14. An der Anzeigewand 13 sind Anzeigeelemente sowie Betätigungsmittel (nicht dargestellt) des vorliegenden Gerätes angebracht.

Die Betätigungseinrichtung 10 umfasst ferner eine Vorrichtung 20 zur Handhabung des Deckels 5 der Ablagerungsvorrichtung 1. Die Handhabungsvorrichtung 20 ist so ausgeführt, dass der Kulturboden 4 während einer gewünschten Zeitspannen für die Aufnahme von Mikroorganismen offen bleiben kann. Dies wird unter anderem dadurch erreicht, dass der Deckel 5 durch die Handhabungsvorrichtung 20 so bewegt wird, dass der Deckel 5 vom Unterteil 3 weg und zu diesem wieder zurück bewegt werden kann.

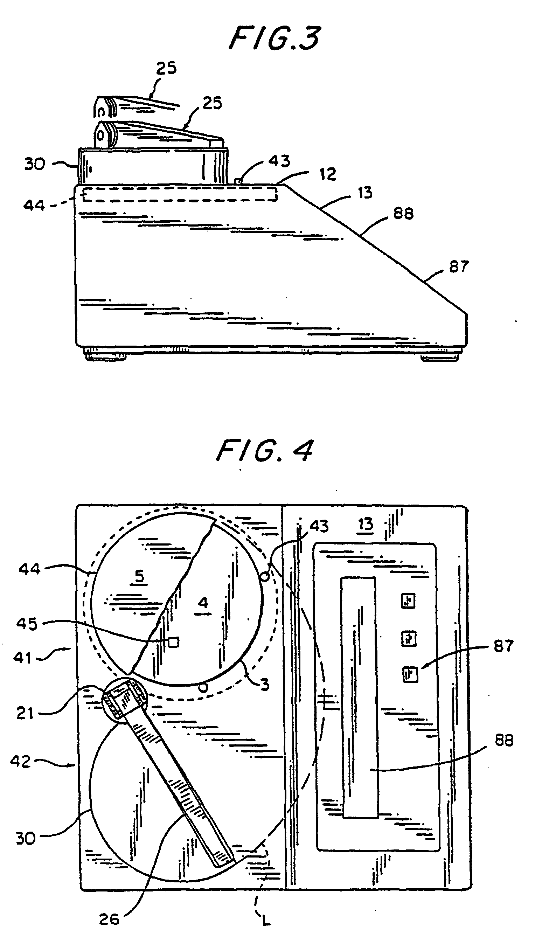

Die Handhabungsvorrichtung 20 weist eine Welle 21 auf, welche praktisch vertikal verläuft und welche durch die Oberwand 12 des Gehäuses 11 hindurchgeht. Dabei ist diese Welle 21 in einem Lager 22 gelagert, welches in der Oberwand 12 des Gehäuses 11 eingesetzt ist. Diese Lagerung ist so ausgeführt, dass die Welle 21 im Lager 22 nicht nur schwenken sondern sich auch in ihrer Achsialrichtung hin und her verschieben kann. An der oberen bzw. sich oberhalb der Oberwand 12 des Gehäuses 11 befindlichen Endpartie der Welle 21 sind Tragmittel 25 für den Deckel 5 der Petrischale 1 angelenkt. Die Verschiebbarkeit der Welle 21 in ihrer Achsialrichtung ist in Fig. 1 und 3 durch zwei übereinander liegende Stellungen der Tragmittel 25 angedeutet.

Die Tragmittel 25 umfassen einen Arm 26, welcher einerends mit Hilfe eines Zapfens 27 am oberen Ende der Welle 21 angelenkt ist. Um diesen Zapfen 27 herum ist der Arm 26 gegenüber der Welle 21 schwenkbar. Die die Schwenkbewegung dieses Armes 26 begrenzenden Anschläge an der Welle 21 sind so ausgebildet, dass der Arm 26 zwischen seiner praktisch horizontalen und seiner praktisch vertikalen Lage schwenken kann. Der Schwenkarm 26 hat einen viereckförmigen Querschnitt, wobei zwei der Seiten 28 und 29 desselben vertikal verlaufen. In Fig. 2 ist der Arm 26 in seiner zurückgezogenen Stellung dargestellt. In dieser Lage des Armes 26 steht der Unterteil 3 der Petrischale 1 frei und Keime oder ähnlich können auf dem Kulturboden 4 sedimentieren. Die erste der genannten Seitenflächen 28 des Armes 26 ist die Vorderseite des Armes 26. Eine Kurvenlinie L zeigt die Bahn eines am freien Ende des Armes 26 liegenden Punktes während des Betriebes dieses Gerätes.

An der Unterseite der freien Endpartie des Schwenkhebels 26 ist ein Träger 30 für den Deckel 5 der Petrischale 1 befestigt. Dieser Träger 30 weist eine horizontal verlaufende Grundplatte 31 auf, welche eine praktisch halbkreisförmige Kontur hat (Fig. 2). Die geradlinig verlaufende Kante 32 der Grundplatte 31 ist der Vorderseite 28 des Schwenkarmes 26 so zugeordnet, dass diese Kante 32 parallel zur Vorderseite 28 des Armes 26 verläuft. Foglich steht der praktisch halbkreisförmige Abschnitt 33 der Grundplatte 31 von der Rückseite 29 des Schwenkarmes 26 ab. Vom halbkreisförmigen Rand 34 der Grundplatte 31 steht eine im wesentlichen vertikal verlaufende und ebenfalls halbkreisförmig verlaufende Seitenwand 35 dieses Trägers 30 herab. An den unteren Rand dieser Seitenwand 25 schliesst sich eine Greifwand 36 an, welche die Form eines Abschnittes eines flachen und schmalen Ringes hat. Diese Greifwand 36 schliesst sich mit ihrem Aussenrand an die untere Randkante der genannte Seitenwand 35 an. Die Innenkante 37 dieser Greifwand 36 steht frei. Die Grundplatte 31, die Seitenwand 35 und die Greifwand 36 bilden zweckmässigerweise ein Stück.

Der Radius der Innekante 37 am Greifring 36 entspricht dem Radius des Unterteiles 3 der Petrischale 1. Der Radius der Seitenwand 35 des Trägers 30 entspricht dagegen dem grössten Radius des Deckels 5 der Petrischale 1, d.h. dem Radius der unteren Kante 7 der herabhängenden Seitenwand 6 des Deckels 5. Die Höhe der Seitenwand 35 des Trägers 30 entspricht der Höhe der genannten Seitenwand 6 des Deckels 5. Der Ausdruck "entspricht" bedeutet hier, dass die genannten Abmessungen des Trägers 30 zumindest so gross sind wie die entsprechenden Abmessungen der Petrischale 1. Die Abmessungen am Träger 30 können etwas grösser sein als die entsprechenden Abmessungen der Petrischale 1, damit der Betrieb des vorliegenden Gerätes reibungslos ablaufen kann. Der Träger 30 muss nämlich so dimensioniert sein, dass er den Deckel 5 problemlos aufnehmen und transportiren kann. Zu diesem Zweck kann die Tiefe bzw. die zum Arm 26 senkrecht stehende Abmessung der Grundplatte 31 der im wesentlichen halbrunden Grundplatte 31 etwas grösser sein als dr Radius der unteren Kante 7 am Deckel 5 der Petrischale 1. Diese zusätzliche Tiefe der Grundplatte 31 kann beispielsweise der Breite des Armes 26, d.h. dem Abstand zwischen der Vorderwand 28 und der Rückwand 29 des Armes 26 entsprechen. Der Radius der Innenkante 37 der Greifwand sowie die Breite dieser Greifwand 36 müssen so sein, dass diese Wand 36 die untere Kante 7 des Deckels 5 der Petrischale 1 zuverlässig untergreift und während der Schwenkbewegung des Trägers 30 zuverlässig hält.

Die Welle 21 ist etwa in der Mitte der Länge der Oberwand 12 des Gerätegehäuses 11 angeordnet. Auf der oberen Seite der Oberwand 12 sind links und rechts von der Welle 21 Aufnahmestationen 41 und 42 für die einzelnen Teile 3 und 5 der Petri-schale 1 ausgeführt. In der ersten Aufnahmestation 41 ist der Unterteil 3 der Petrischale 1 untergebracht und mit Hilfe von Positionsstiften 43 an Ort und Stelle gehalten. Diese erste Aufnahmestation 41 umfasst ferner eine elektrothermische Vorrichtung 44 (Fig. 3 und 4), welche sich vorteilhaft unterhalb der Oberwand 12 befindet. Mit Hilfe dieser elektrothermischen Vorrichtung 44 kann der Unterteil 3 der Petrischale 1 samt dem Kulturboden 4 und den auf diesem sedimentierten Keimen je nach Situation entweder erwärmt (bebrütet) werden, wenn die Umgebungstemperatur niedrig ist, oder gekühlt werden, wenn die Umgebungstmperatur zu hoch ist. Diese Vorrichtung 44 kann ein sogenanntes und an sich bekanntes Peltier-Element enthalten.

In Fig. 2 ist der Träger 30 samt dem Deckel 5 in der zweiten Aufnahmestation 42 dargestellt, sodass der Deckel 5 sich in seiner vom Unterteil 3 entfernten Stellung befindet. Bei dieser Stellung des Deckels 5 können Keime auf dem Kulturboden 4 im Unterteil 3 sedimentieren. In Fig. 4 ist der Deckel 5 im Bereich der ersten Aufnahmestation 41 auf dem Unterteil 3 aufgesetzt dagestellt, sodass keine weiteren Keime mehr zum Kulturboden 4 gelangen können. Der Träger 30 ist in seiner zurückgezogegenen Stellung im Bereich der zweiten Aufnahmestation 42 dargestellt, sodass der Deckel 5 frei ist. Vom Deckel 5 ist in Fig. 4 nur ein Abschnitt desselben abgebildet, damit ein Einblick in das Innere der Petrischale 1 mit dem Kulturboden 4 möglich ist. Auf dem in Fig. 4 abgebildeten Abschnitt des Kulturbodens 4 ist eine Kolonie 45 angedeutet. Diese ergab sich aus einem Keim, welcher zum Kulturboden gelangte, während der Unterteil 3 unverdeckt war.

Im Inneren des Gehäuses 11 und unterhalb der Oberwand 12 des Gehäuses 11 ist eine Hilfsplatte 50 angeordnet, welche mit Hilfe von Distanzstücken 51 an der Unterseite der Oberwand 12 des Gehäuses 11 befestigt ist. Diese Hilfsplatte 50 verläuft praktisch parallel zur Oberwand 12 und sie trägt unter anderem ein zweites Lager 52 für die untere Endpartie der Welle 21. Dieses zweite Lager 52 ist mit dem ersten Lager 22 axial ausgerichtet. Die untere Endpartie der Welle 21 ist so lang ausgeführt, dass sie aus dem zweiten bzw. unteren Lager 52 heraus ragt.

Auf der Oberseite der Hilfsplatte 50 ist eine Steuerscheibe 55 drehbar gelagert, deren Umfangspartie mit Zähnen 56 versehen ist. Dieser Umfangspartie der Steuerscheibe 55 ist ein Antriebsmotor 57 zugeordnet, dessen Ritzel 58 mit den Zähnen 56 an der Steuerscheibe 55 kämmt.

Von der Unterseite der Hilfsplatte 50 steht zumindest ein Lagerbock 59 ab, an welchem ein zweiarmiger Winkelhebel 60 schwenkbar gelagert ist. Der Winkel zwischen den Armen 61 und 62 dieses Winkelhebels 60 beträgt etwa 90 Grad. Das freie Ende des ersten Armes 61 des Winkelhebels 60 ist am unteren Ende der Welle 21 mit Hilfe eines Zapfens 68 angelenkt. Der zweite Arm 62 des Winkelhebels 60 geht durch eine entsprechende Oeffnung in der Hilfsplatte 50 hindurch, sodass die freie Endpartie des zweiten Armes 62 des Winkelhebels 60 im Bereich der Steuerscheibe 55 liegt, und zwar unterhalb dieser. Das freie Ende des zweiten Armes 62 des Winkelhebels 60 ist mit einer Rolle 63 versehen. Die Oberfläche dieser Rolle 63 hat die Form der Oberfläche einer Kugelschicht, bei der sich der Aequator der Kugel in der Mitte der Höhe dieser Kugelschicht befindet.

In jenem Bereich der Welle 21, welcher sich zwischen den zwei Lagern 22 und 52 befindet, ist die Welle 21 mit einem Zahnkranz 64 versehen, welcher die Welle 21 umgibt und welcher mit der Welle 21 fest verbunden ist. Es ist eine horizontal angeordnete und verschiebbare Zahnstange 65 vorgesehen, welche sich oberhalb der Steuerscheibe 55 befindet und deren Zähne 66 mit dem Zahnkranz 64 an der Welle 21 kämmen. Die Unterseite der Zahnstange 65 trägt eine Führungsrolle 67, welche praktisch rechtwinklig vom Grundkörper der Zahstange 65 absteht.

In der oberen und der unteren grossflächigen Seite der Steuerscheibe 55 ist je eine Steuerkurve 71 bzw. 72 ausgeführt. Im dargestellten Fall sind diese Steuerkurven 71 und 72 als Vertiefungen bzw. Rillen in den grossflächigen Seiten des Grundkörpers der Steuercheibe 55 ausgeführt, welche einen im wesentlichen viereckförmigen Querschnitt aufweisen. Die untere Steuerkurve 71 dient zur Steuerung der vertikalen Bewegung der Tragmittel 25 für den Deckel 5 der Petrischale 1. In dieser Steuerkrve 71 liegt die Rolle 63 des zweiten Armes 62 des Winkelhebels 60. Die obere Steuerkurve 72 dient zur Steuerung der Schwenkbewegungen des Trägers 30 der Tragmittel 25 in horizontaler Richtung. In dieser Steuerkurve 72 Liegt die Führungsrolle 67 an der Zahnstange 65.

Die erste Steuerkurve 71 hat im wesentlichen die Form einer Spirale mit einer einzigen Windung. Wenn die Steuerscheibe 55 eine Umdrehung ausführt, verschiebt sich die in dieser Steuerkurve 71 liegende Rolle 63 am zweiten Arm 62 des Winkelhebels 60 in der Radialrichtung der Steuerscheibe 55. Das an die Welle 21 angeschlossene Ende des ersten Armes 61 dieses Winkelhebels 60 bewegt dabei die Welle 21 in vertikaler Richtung. Je nach der Drehrichtung der Steuerscheibe 55 bewegt sich die Welle 21 entweder aufwärts oder abwärts. Die zweite Steuerkurve 72 ist unrund und sie weist Abschnitte auf, welche eine hin und her gehende Horizontalbewegung des Trägers 30 des Deckels 5 oder einen Stillstand dieses Trägers 30 ermöglichen. Während dieser Stillstandsabschnitte wird der Träger 30 mittels der ersten Steuerkurve 71 in vertikaler Richtung bewegt, wie dies vorstehend beschrieben ist.

Die beschriebenen Massnahmen ermöglichen, dass die Tragmittel 25 zugleich zwei Bewegungen ausführen, welche dazu erforderlich sind, um den Deckel 5 der Petrischale zwischen der ersten Station 41 und der zweiten Station 42 zu bewegen. Die genannten Massnahmen ermöglichen auch, den Deckel 5 in der ersten Station 41 auf den Unterteil 3 der Petrischele 1 aufzusetzen. Hiernach wird der Träger 30 ohne den Deckel 5 und wiederum entlang der Kurve L in die zweite Station 42 zurückbewegt bis er jene Stellung einnimmt, welche in Fig. 2 dargestellt ist.

Eine Petrischale 1, bei welcher ihr Deckel 5 auf ihrem Unterteil 3 aufgesetzt ist, wird von Hand in die erste Station 41 des vorliegenden Gerätes gebracht. Nachdem einer der Arbeitsgänge gewählt worden ist, bewegt sich der Träger 30 für den Deckel 5 der Petrischale 1 entlang der Kurve L aus der zweiten Station 42 in die erste Station 41, wobei dieser Träger 30 sich in seiner unteren Stellung befinden. Diese Horizontalbewegung endet, wenn die Vorderkante 37 der Greifwand 36 am Träger 30 in die Nähe des Unterteiles 3 der Petrischale 1 gelangt oder die Seitenwand dieses Unterteils 3 sogar berührt. Hierbei kommt die Greifwand 36 des Trägers 30 unter den unteren Rand 7 der Seitenwand 6 des Deckels 5 zu Liegen. Jetzt wird die Welle 21 aufwärts bewegt, sodass der Deckel 5 dank der unter der Deckelwand 6 leigenden Greifwand 36 des Trägers 30 vom Unterteil 3 abgehoben wird. Hiernach bewegt sich der Träger 25 samt dem Deckel 5 zurück in die zweite Station 42, wobei er in seiner oberen vertikalen Lage befindet.

Nach Ablauf einer Zeitspanne, welche für die Sedimantation von Keimen auf dem Kulturboden 4 vorgesehen ist, bewegt sich der Träger 25 samt dem Deckel 5 gegen die erste Station 41 hin, wobei er sich immer noch in seiner oberen Stellung befindet. In der ersten Station 41 wird der Träger 30 abgesenkt, wodurch der Deckel 5 auf dem Unterteil 3 der Petrischale 1 abgesezt wird. In dieser unteren Stellung bewegt sich der Träger 25 jetzt in die zweite Station 42 zurück.

Nach Verschliessen der Petrischale 1 können Keime, welche während der Oeffnungszeit der Petrischle 1 auf dem Kulturboden 4 sedimentiert haben, zu Kolonien 45 wachsen. Nach einer vorgegebenen Zeitspanne zählt mann die Anzahl der genannten Kolonien, woraus man auf die Anzahl von Mikroorganismen in der Luft schliessen kann.

Damit diese und noch weitere Arbeitschritte automatisch ausgeführt werden können, ist das Gerät mit einer Steuervorrichtung 80 ausgerüstet. Diese Steuervorrichtung 80 ist im Gehäuse 11 des Gerätes untergebracht und sie enthält eine Steuereinheit 81, welche einen Mikroprozessor 82, eine Regeleinheit 83, einen Timer 84 sowie einen EEPROM 85 umfasst. Diese Steuereinheit 81 wird mittels einer Speisung 86 mit elektrischem Strom versorgt, welcher auch zur Speisung des Motors 57 verwendet wird. Die jeweilige Betriebsart des Gerätes wird mit Hilfe von Tasten 87 eingegeben, welche sich im Bereich der Anzeigewand 13 des Gerätegehäuses 11 samt an sich bekannten Anzeigemitteln 88 befinden. Für den automatischen Ablauf ist es erforderlich, Fühler 89 zur Feststellung der Position des Trägers 30 vorzusehen. Diese sind an betreffenden Stellen der Bahn L des Trägers 30 angeordnet. Ferner umfasst die Steuervorrichtung 80 auch Temperaturfühler 90, welche im Bereich der ersten Station 41 angebracht sind.

Im EEPROM 85 sind unter anderem Programme zur Handhabung der Petrischale 1 gespeichert. Diese Programme können verschiedene Bedingungen, wie z.B. Zeit, Temperatur usw. für die Bebrütung des Kulturbodens 4 mit den sedimentierten Mikroorganismen enthalten. Zusammen mit der Wahl von geeigneten Kulturboden 4 können solche Programme zu einer selektiven Erfassung von Mikroorganismen verwendet werden.

Da die Menge von Mikroorganismen in der Luft auch von der Höhe über dem Boden abhängt, ist es zweckmässig, einen Stativ (nicht dargestellt) vorzusehen, auf welchem das Gehäuse 11 des Gerätes befestigt ist. Um die Dichte der Keime in verschiedenen Höhen messen zu können, ist der Stativ so ausgeführt, dass seine Höhe verstellbar ist.

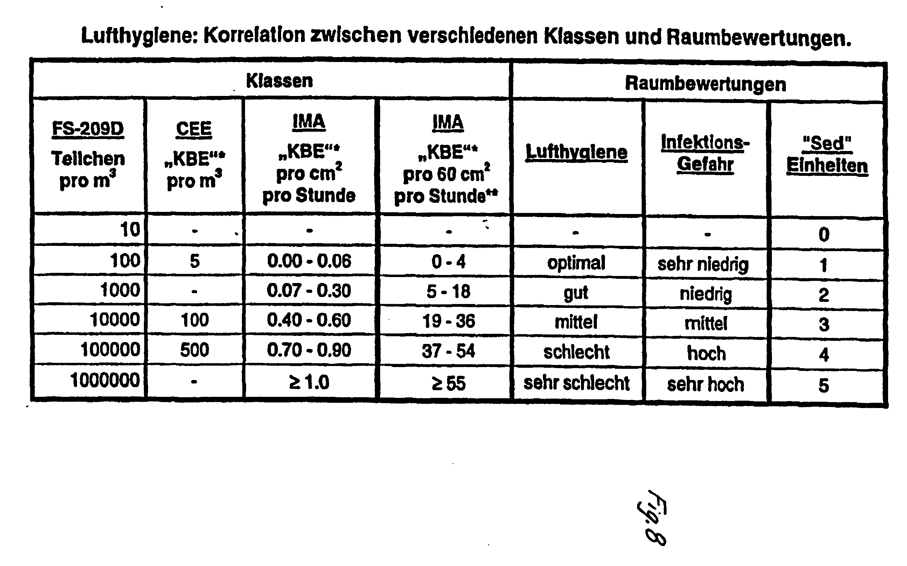

Fig. 8 zeigt eine Tabelle, in welcher unter anderem aufgezeigt ist, wie man die hygienische Situation in Räumen bisher bewertet hat. Die Angaben in der ersten Spalte der Tabelle sind zwar verhältnismässig klar, aber sie verwenden grosse Zahlen, was in der Praxis unhandlich ist. Die Bewertung (CEE) in der zweiten Spalte der Tabelle in Fig. 8 umfasst nicht alle Klassen der Lufthygiene. Die "IMA"-Spalten arbeiten mit Angaben von Bereichen, was ebenfalls unhandlich ist. Die in Worten gefasste Bewertung in den zwei vorletzeten Spalten ist ungenau.

Im vorliegenden Fall werden den einzelnen Klassen der Raumbewertung Zahlen von 1 bis 5 zugeordnet, welche mit den in der dritten Spalte der Tabelle aus Fig. 8 angegebenen Mengen von Teilchen pro cm2 und pro Stunde in einer direkten Beziehung stehen.

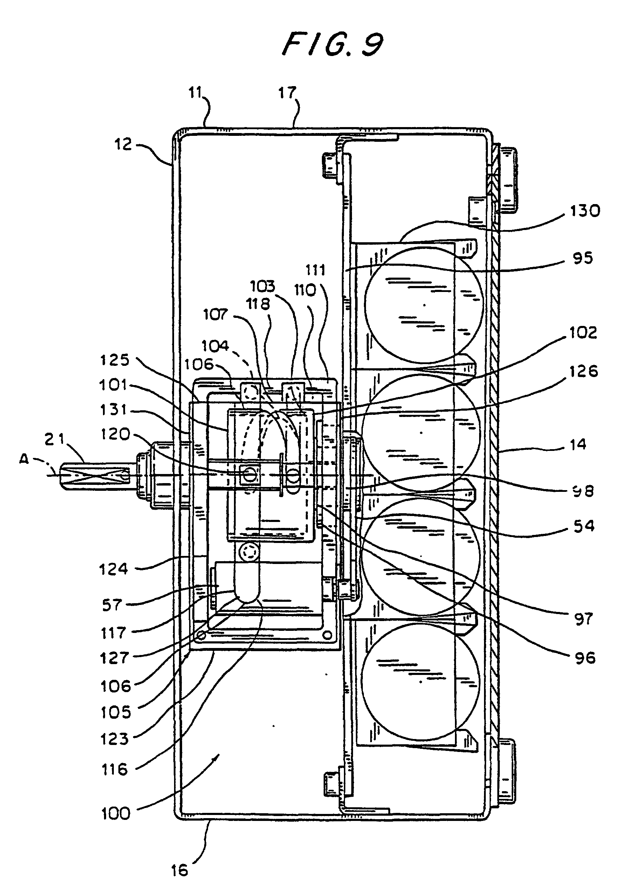

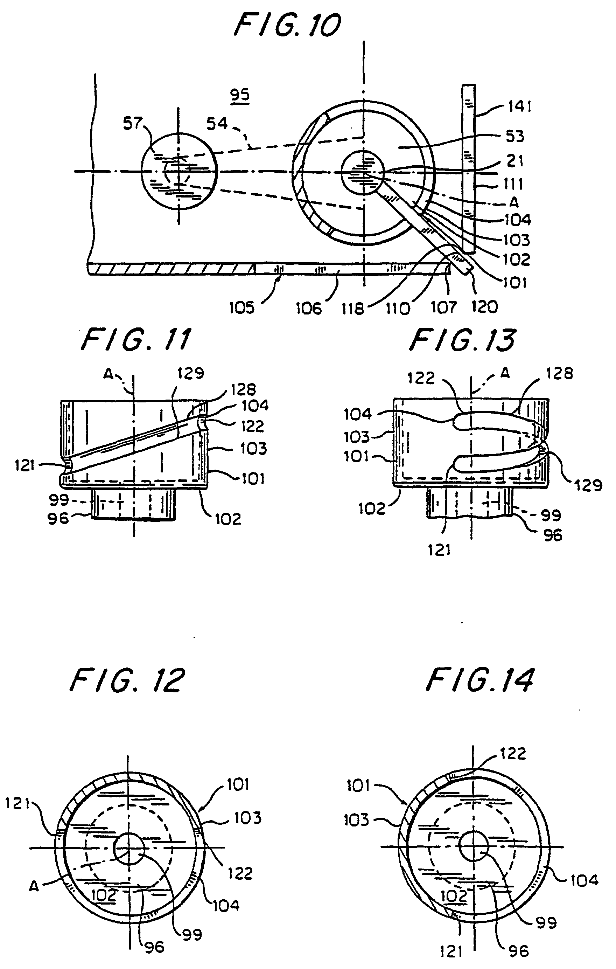

Fig. 9 zeigt eine weitere Ausführungsmöglichkeit des vorliegenden Gerätes, und zwar in einer Frontansicht, nachdem die Frontwand dieses Gerätes entfernt worden ist. Im Gehäuse 11 des Gerätes ist eine Tragplatte 95 angeordnet, auf welcher eine zweite Ausführungsmöglichkeit der Einrichtung 100 zur Betätigung der Ablagerungsvorrichtung 1 montiert ist. Fig. 10 zeigt in Draufsicht die wichtigsten Bestandteile dieser Einrichtung 100.

Die Einrichtung 100 umfasst eine Kulisse 101, welche auf der Tragplatte 95 drehbar bzw. schwenkbar gelagert ist. Im dargestellten Fall ist diese Kulisse 101 im wesentlichen becher-bzw. hülsenförmig und sie weist einen Boden 102 (Fig. 11 bis 14) auf, von dem weg sich eine zylinderförmige Seitenwand 103 aufwärts erstreckt. Die Drehachse dieser Kulisse 101 verläuft im dargestellten Fall vertikal und sie fällt mit der Hauptachse A dieser Einrichtung 100 zusammen.

An die Unterseite des Kulissenbodens 101 schliesst sich eine hohle Welle 96 an, welche in der Mitte des Kulissenbodens 102 angeordnet ist und welche die Kulisse 101 trägt. Diese Tragwelle 96 ist in einem Lager 97 drehbar bzw. schwenkbar gelagert. Durch die Oeffnung 99 in der Tragwelle 96 geht die Schwenkwelle 21 hindurch uns sie ist in dieser Oeffnung 99 sowohl schwenkbar als auch längsverschiebbar gelagert. Die Achse der Tragwelle 96 fällt mit der Hauptachse A dieser Einrichtung 100 ebenfalls zusammen. Das Lager 97 ist in der Tragplatte 95 eingelassen, sodass ein Teil der Tragwelle 96 von der Unterseite des Lagers 97 abstehen kann. Dieser Abschnitt der Tragwelle 96 ist mit einer Scheibe 98 versehen, welche über die Tragwelle 96 mit der Kulisse 101 fest verbunden ist. Diese Scheibe 98 ist über einen Riemen 54 an den Antriebsmotor 57 dieser Einrichtung 100 angeschlossen.

In der Wand 103 der Kulisse 101 ist eine Nut 104 ausgeführt, welche einen Durchbruch in der Kulissenwand 103 darstellt. Diese Nut 104 verläuft hinsichtlich der Achse A schräg. Dies bedeutet, dass das eine Ende 121 der Nut 104 tiefer bzw. näher zum Kulissenboden 102 liegt als das andere Ende 122 dieser Nut 104. Die Winkellänge der Nut 104 beträgt mehr als 180 Grad und sie beträgt vorteilhaft 210 Grad. Die Kulissennut 104 weist eine obere Längskante 128 und eine untere Längskante 129 auf, welche sich zwischen den Enden 121 und 122 der Nut 104 erstrecken und stetig verlaufen.

Die Einrichtung 100 umfasst ferner eine Führungsplatte 105, welche neben der Kulisse 101 angeordnet ist und welche praktisch parallel zu einer Ebene verläuft, in welcher die Hauptachse A dieser Einrichtung 100 liegt. Die Führungsplatte 105 befindet sich in einem Abstand von der Aussenseite der Kulisse 101. Im dargestellten Fall ist der Grundkörper 131 der Platte 105 im wesentlichen rechteckförmig, wobei die längeren Seiten 124 und 126 dieses Rechteckes 131 horizontal verlaufen. Die kürzeren Seiten 123 und 125 des Rechteckes 131 verlaufen vertikal.

Die Führungsplatte 105 weist einen geradlinigen Schlitz 106 auf, welcher praktisch horizontal, d.h. parallel zu den längeren Seiten 124 und 126 der Führungsplatte 105 verläuft und praktisch auf derselben Höhe liegt wie die obere Endpartie 122 der Nut 104 in der Kulisse 101. Der Schlitz 106 ist gegenüber der Schwenkwelle 21 so angeordnet, dass die Schwenkwelle 21 etwa in der Mitte der Länge des Schlitzes liegt. Eines der Enden dieses Schlitzes 106 liegt in einer der Seiten 125 des Grundkörpers 131 der Führungsplatte 105, sodass der Schlitz 106 sich hier öffnet und sich an diese Seite 125 der Führungsplatte 106 anschliesst. Das andere Ende 127 des Schlitzes 106 ist dagegen durch einen Boden geschlossen. Der Schlitz 106 umfasst ferner eine untere Kante 116 und eine obere Kante 117, welche ebenfalls horizontal, d.h. parallel zu den Längsseiten 124 und 126 der Führungsplatte 105 verlaufen.

Die Platte 105 weist ferner eine Führungskante 107 auf. Diese Kante 107 stellt einen Abschnitt jener Seite 125 der Platte 105 dar, in welcher das offene Ende des Schlitzes 106 liegt, wobei die Führungskante 107 sich zwischen diesem offenen Ende des Schlitzes 106 und der Unterseite 126 der Führungsplatte 105 erstreckt. Diese Führungskante 107 verläuft somit praktisch parallel zur Hauptachse A dieser Einrichtung 100.

Die Einrichtung 100 umfasst auch einen Anschlag 111, welcher im dargestellten Fall einen im wesentlichen plattenförmigen Grundkörper 141 aufweist. Dieser plattenförmige Grundkörper 141 verläuft parallel zu einer Ebene, in welcher die Hauptachse A der Einrichtung 100 liegt, wobei er etwa rechtwinklig zur Führungsplatte 105 steht. Die der Führungsplatte 105 am nächsten liegende Kante 118 der Anschlagplatte 111 ist der Führungskante 107 der Führugsplatte 105 zugeordnet, und zwar derart, dass ein Spalt zwischen diesen Kanten 107 und 118 frei vorhanden ist. Die Anschlagkante 118, obwohl sie parallel zur Führungskante 107 verläuft, ist länger als die Führungskante 107. Das obere Ende der Anschlagkante 118 liegt höher als das obere Ende der Führungskante 107, sodass ein Abschnitt der Anschlagkante 118 der offenen Mündung des Schlitzes 106 in der Führungsplatte 105 gegenübersteht.

Ein Bolzen 120 ist an der Welle 21 einerends befestigt und er steht praktisch rechtwinklig von dieser Welle 21 ab. Der Bolzen 120 ist an der Welle 21 so angeordnet und er ist so lang ausgeführt, dass er durch die Nut 104 in der Kulisse 101 hindurchgehen kann und dass seine freie Endpartie im Bereich der Führungsplatte 105 liegt. Der Querschnitt des Bolzens 120 ist kreisförmig. Die Breite der Nut 104, des Schlitzes 106 und des bereits erwähnten Spaltes zwischen der Führungskante 107 und der Anschlagkante 118 entspricht dem Durchmesser des Bolzens 120, und zwar derart, dass der Bolzen 120 sich in der Nut 104, im Schlitz 120 und im genannten Spalt möglichst reibungsarm bewegen kann.

Bei der Beschreibung der Arbeitsweise dieser Einrichtung 100 kann man von der Situation ausgehen, dass der Bolzen 120 sich unten in der Kulissennut 104 und im Spalt zwischen den Kanten 107 und 118 befindet. Dabei ist der Deckel der Petrischale auf dem Unterteil derselben aufgesetzt. Wird die Kulisse 101 mittels des Antriebes 57 im Uhrzeigersinn gedreht, so schlägt der Bolzen 120 an der Führungskante 107 an und wegen dem Anstieg der Kulissennut 104 bewegt sich der Bolzen 120 entlang dieser Führungskante 107 aufwärts, bis er die Höhe der offenen Mündung des Schlitzes 106 in der Führungsplatte 105 erreicht hat. Während dieser Phase führt die mit dem Bolzen 120 gekoppelte Schwenkwelle 21 eine aufwärts gerichtete Hubbewegung aus und der Deckel der Petrischale wird dabei vom Unterteil der Petri-schale abgehoben.

Am Ende der Hubbewegung steht der Bolzen 120 dem offenen Ende des Schlitzes 106 in der Führungsplatte 105 gegenüber. Da der Antrieb 57 die Kulisse 101 jedoch in derselben Richtung weiter antreibt, drückt der Boden des oberen Endes 122 der Kulissennut 104 den Bolzen 120 in den Führungsschlitz 106 hinein und bewegt diesen im Schlitz 106, bis der Bolzen 120 am Schlitzboden 127 anschlägt. Während dieser Phase hat die Welle 21 eine Schwenkbewegung ausgeführt und dabei ist der Deckel der Petrischale vom Unterteil entfernt worden.

Wird die Drehrichtung des Antriebes 57 jetzt umgekehrt, dann bewegt sich die Kulisse 101 im Gegenuhrzeigersinn. Der Bolzen 120 wird durch die obere und schräg abfallende Kante 128 der Kulissennut 104 im Schlitz 106 gegen das offennes Ende dieses Schlitzes 106 hin gedrückt. Der Bolzen 120 bewegt sich im Schlitz 106, bis er an der diesem Schlitzende gegenüberstehenden Anschlagkante 118 anschlägt. Die Welle 21 wurde während dieser Bewegung des Bolzens 120 zurückgeschwenkt, sodass der Deckel der Petrischlasle zum Unterteil derselben zurückgebracht wurde.

Die Kulisse 101 wird durch den Antrieb 57 weiter in derselben Richtung geschwenkt bzw. gedreht. Der Bolzen 120 befindet sich dabei im Anschlag an der Anschlagkante 118, sodass er sich in der horizontalen Richtung nicht weiter bewegen kann. Unter dem Bolzen 120 befindet sich jetzt jedoch der genannte Spalt zwischen den Kanten 107 und 118 und der Bolzen 120 wird durch die absteigende Nut 104 in diesen Spalt gedrückt. Dabei führt die Welle 21 eine abwärts gerichtete Hubbewegung aus und der Deckel der Petrischale wird auf den Unterteil derselben aufgesetzt.

## Patentansprüche

1. Gerät zur Bestimmung der Zahl von Mikroorganismen in Luft, mit einer Vorrichtung (1) zur Ablagerung der Mikroorganismen, wobei diese Vorrichtung (1) einen Unterteil (3) und einen Deckel (5) aufweist, mit einem Nähr- bzw. Kulturboden (4), welcher sich im Inneren der Ablagerungsvorrichtung (1) befindet, und mit einer Einrichtung (10) zur Betätigung des Deckels (5) der Ablagerungsvorrichtung (1), **dadurch gekennzeichnet, dass** das Gerät eine erste Station (41) aufweist, die zur Aufnahme des Unterteiles (3) der Ablagerungsvorrichtung (1) bestimmt ist, dass das Gerät eine zweite Station (42) aufweist, welche zur Aufnahme des Deckels (5) der Ablagerungsvorrichtung (1) bestimmt ist, dass diese Aufnahmestationen (41,42) sich nebeneinander und auf praktisch derselben Ebene befinden, dass die Betätigungseinrichtung (10) eine Vorrichtung (20) zur Handhabung des Deckels (5) aufweist, dass diese Handhabungsvorrichtung (20) eine Welle (21) aufweist, welche durch die genannten Ebene hindurchgeht, dass diese Welle (21) zwischen den Aufnahmestationen (41,42) angeordnet ist und dass die Betätigungseinrichtung (10) so ausgeführt ist, dass der Deckel (5) zwischen den zwei Aufnahmestationen (41,42) in gesteuerter Weise bewegt werden kann.

2. Gerät nach Patentanspruch 1, **dadurch gekennzeichnet, dass** die Betätigungseinrichtung (10) femer eine Vorrichtung (80) zur Steuerung der Bewegungen des Deckels (5) umfasst.

3. Gerät nach Patentanspruch 1, **dadurch gekennzeichnet, dass** die Handhabungsvorrichtung (20) Tragmittel (25) für den Deckel (5) aufweist, dass die Tragmittel (25) einer ersten Endpartie der Welle (21) der Handhabungsvorrichtung (20) zugeordnet sind und dass Mittel zur mechanischen Bewegung der Welle (21) der anderen bzw. zweiten Endpartie dieser Welle (21) zugeordnet sind.

4. Gerät nach Patentanspruch 3, **dadurch gekennzeichnet, dass** die Tragmittel (25) einen Arm (26) umfassen, dass dieser Arm (26) einerends an die erste Endpartie der Welle (21) angeschlossen ist und dass ein Träger (30) für den Deckel (5) der Ablagerungsvorrichtung (1) am anderen bzw. freien Ende des Armes (26) angebracht ist.

5. Gerät nach Patentanspruch 3, **dadurch gekennzeichnet, dass** die Bewegungsmittel einen durch eine Steuervorrichtung (80) steuerbaren Motor (57) umfassen, dass die Bewegungsmittel femer eine Steuerscheibe (55) umfassen, welche durch den Motor (57) antreibbar ist, dass diese Scheibe (55) mit Steuerkurven (71,72) versehen ist, und dass die Bewegungsmittel auch einen Hebel (60) sowie eine Zahnstange (65) umfassen, welche einerseits mit den Steuerkurven (71,72) an der Steuerscheibe (55) und andererseits mit der Welle (21) gekoppelt sind.

6. Gerät nach Patentanspruch 4, **dadurch gekennzeichnet, dass** der Arm der Tragmittel (25) mit Hilfe eines Zapfens (27) am oberen Ende der Welle (21) angelenkt ist und dass Anschläge an der Welle (21) ausgebildet sind, welche die Schwenkbewegungen des Armes (26) begrenzen, sodass der Arm (26) zwischen einer praktisch horizontalen und einer praktisch vertikalen Lage schwenken kann.

7. Gerät nach Patentanspruch 4, **dadurch gekennzeichnet, dass** der Träger (30) für den Deckel (5) der Petrischale (1) an der Unterseite der freien Endpartie des Schwenkhebels (26) angebracht ist, dass dieser Träger (30) eine horizontal verlaufende Grundplatte (31) aufweist, welche eine praktisch halbkreisförmige Kontur hat, dass die geradlinig verlaufende Kante (32) der Grundplatte (31) der Vorderseite (28) des Schwenkarmes (26) so zugeordnet ist, dass diese Kante (32) parallel zur Vorderseite (28) des Armes (26) verläuft und der praktisch halbkreisförmige Abschnitt (33) der Grundplatte (31) von der Rückseite (29) des Schwenkarmes (26) absteht, dass eine im wesentlichen vertikal verlaufende und ebenfalls halbkreisförmig verlaufende Seitenwand (35) dieses Trägers (30) vom halbkreisförmigen Rand (34) der Grundplatte (31) herabhängt, dass sich an den unteren Rand der Seitenwand (35) eine Greifwand (36) an schliesst, welche die Form eines Abschnittes eines flachen und schmalen Ringes hat, dass sich diese Greifwand (36) mit ihrem Aussenrand an die untere Randkante der genannten Seitenwand (35) an schliesst, dass der Radius der Innenkante (37) am Greifring (36) mit dem Radius des Unterteiles (3) der Petrischale (1) entspricht, dass der Radius der Seitenwand (35) des Trägers (30) dagegen dem grössten Radius des Deckels (5) der Petrischale (1), d.h. dem Radius der unteren Kante (7) der herabhängenden Seitenwand (6) des Deckels (5) entspricht und dass die Höhe der Seitenwand (35) des Trägers (30) der Höhe der genannten Seitenwand (6) des Deckels (5) entspricht.

8. Gerät nach Patentanspruch 3, **dadurch gekennzeichnet, dass** die Betätigungseinrichtung (10) ein Gehäuse (11) umfasst, welches eine horizontal verlaufende Oberwand (12) aufweist, dass die Welle (21) in einem Lager (22) gelagert, welches in der Oberwand (12) des Gehäuses (11) eingesetzt ist, dass das Lager (22), so ausgeführt ist, dass die Welle (21) im Lager (22) nicht nur schwenken sondern auch in ihrer Achsialrichtung hin und her verschoben werden kann, dass die Tragmittel (25) für den Deckel (5) der Petrischale (1) an der oberen bzw. sich oberhalb der Oberwand (12) des Gehäuses (11) befindlichen Endpartie der Welle (21) angelenkt, sind.

9. Gerät nach Patentanspruch 8, **dadurch gekennzeichnet, dass** im Inneren des Gehäuses (11) und unterhalb der Oberwand (12) des Gehäuses (11) eine Hilfsplatte (50) angeordnet ist, welche mit Hilfe von Distanzstücken (51) an der Unterseite der Oberwand (12) des Gehäuses (11) befestigt sein kann, dass diese Hilfsplatte (50) praktisch parallel zur Oberwand (12) verläuft und unter anderem ein zweites Lager (52) für die untere Endpartie der Welle (21) trägt, dass dieses zweite Lager (52) mit dem ersten Lager (22) axial ausgerichtet ist, dass die untere Endpartie der Welle (21) so lang ausgeführt ist, dass sie aus dem zweiten bzw. unteren Lager (52) herausragt, dass auf der Oberseite der Hilfsplatte (50) eine Steuerscheibe (55) drehbar gelagert ist, deren Umfangspartie mit Zähnen (56) versehen ist, dass dieser Umfangspartie der Steuerscheibe (55) ein Antriebsmotor (57) zugeordnet ist, dessen Ritzel (58) mit den Zähnen (56) an der Steuerscheibe (55) kämmt.

10. Gerät nach Patentanspruch 9, **dadurch gekennzeichnet, dass** von der Unterseite der Hilfsplatte (50) zumindest ein Lagerbock (59) absteht, an welchem ein zweiarmiger Winkelhebel (60) schwenkbar gelagert ist, dass der Winkel zwischen den Armen (61,62) dieses Winkelhebels (60) etwa 90 Grad beträgt, dass das freie Ende des ersten Armes (61) des Winkelhebels (60) am unteren Ende der Welle (21) mit Hilfe eines Zapfens (68) angelenkt ist, dass der zweite Arm (62) des Winkelhebels (60) durch eine entsprechende Oeffnung in der Hilfsplatte (50) hindurchgeht, sodass die freie Endpartie des zweiten Armes (62) des Winkelhebels (60) im Bereich der Steuerscheibe (55) liegt, und zwar unterhalb dieser, dass das freie Ende des zweiten Armes (62) des Winkelhebels (60) mit einer Rolle (63) versehen ist, dass die Oberfläche dieser Rolle (63) die Form der Oberfläche einer Kugelschicht hat, bei der sich der Äquator der Kugel in der Mitte der Höhe dieser Kugelschicht befindet.

11. Gerät nach Patentanspruch 3, **dadurch gekennzeichnet, dass** in jenem Bereich der Welle (21), welcher sich zwischen den zwei Lagem (22) und (52) befindet, die Welle (21) mit einem Zahnkranz (64) versehen ist, welcher die Welle (21) umgibt und welcher mit der Welle (21) fest verbunden ist, dass eine horizontal angeordnete und verschiebbare Zahnstange (65) vorgesehen ist, welche sich oberhalb der Steuerscheibe (55) befindet und deren Zähne (66) mit dem Zahnkranz (64) an der Welle (21) kämmen, dass die Unterseite der Zahnstange (65) eine Führungsrolle (67) trägt, welche praktisch rechtwinklig vom Grundkörper der Zahnstange (65) absteht.

12. Gerät nach Patentanspruch 10, **dadurch gekennzeichnet, dass** in der oberen und in der unteren grossflächigen Seite der Steuerscheibe (55) je eine Steuerkurve (71,72) ausgeführt ist, dass diese Steuerkurven (71,72) als Vertiefungen bzw. Rillen ausgeführt sein können, welche einen im wesentlichen viereckförmigen Querschnitt aufweisen, dass die erste Steuerkurve (71) im wesentlichen die Form einer Spirale mit einer einzigen Windung hat, dass die Rolle (63) des zweiten Armes (62) des Winkelhebels (60) in der unteren Steuerkurve (71) liegt, dass die zweite Steuerkurve (72) unrund ist und Abschnitte aufweist, welche eine hin und her gehende Horizontalbewegung des Trägers (30) des Deckels (5) oder einen Stillstand dieses Trägers (30) ermöglichen, dass die Führungsrolle (67) an der Zahnstange (65) in der oberen Steuerkurve (72) liegt.

13. Gerät nach Patentanspruch 1, **dadurch gekennzeichnet, dass** die Betätigungseinrichtung (100) eine Kulisse (101) umfasst, welche im wesentlichen becher- bzw. hülsenförmig ist, dass diese Kulisse (101) einen Boden (102) und eine sich davon weg erstreckende zylinderförmige Seitenwand (103) aufweist, dass in dieser Seitenwand (103) eine schraubenlinienförmig verlaufende Nut (104) ausgeführt ist, dass die Schwenkwelle (21) der Handhabungsvorrichtung (20) durch eine Oeffnung (99) im Boden (102) der Kulisse (101) hindurchgeht und in dieser Oeffnung (99) sowohl schwenkbar als auch längsverschiebbar gelagert ist, dass die Betätigungseinrichtung (100) femer eine Führungsplatte (105) umfasst, dass diese Führungsplatte (105) eine vertikal verlaufende Führungskante (107) und einen sich an das oben liegende Ende der Führungskante (107) anschliessenden und praktisch horizontal verlaufenden Schlitz (06) aufweist, dass die Betätigungseinrichtung (100) auch einen im wesentlichen plattenförmigen Anschlag (111) umfasst, wobei ein Spalt zwischen der Führungskante (107) der Führungsplatte (105) und der der Führungsplatte (105) am nächsten liegende vertikalen Kante (118) der Anschlagplatte (111) vorhanden ist, dass ein Bolzen (120) an der Welle (21) einerends befestigt ist und von dieser Welle (21) absteht, dass dieser Bolzen (120) an der Welle (21) so angeordnet ist, dass er durch die Nut (104) in der Kulisse (101) hindurchgehen kann, und dass der Bolzen (120) so lang ausgeführt ist, dass seine freie Endpartie im genannten Spalt liegt.

14. Gerät nach Patentanspruch 13, **dadurch gekennzeichnet, dass** sich eine hohle Welle (96) in der Mitte des Kulissenbodens (102) befindet, wobei die Oeffnung in dieser Welle (96) mit der Oeffnung (99) im Kulissenbodens (102) übereinstimmt, dass die Tragwelle (96) in einem Lager (97) drehbar bzw. schwenkbar gelagert ist, dass dieses Lager (97) in einer Tragplatte (95) des Gerätegehäuses eingesetzt ist, sodass ein Teil der Tragwelle (96) von der Unterseite des Lagers (97) absteht, dass dieses freie Ende der Tragwelle (96) mit einer Scheibe (98) versehen ist, welche vorteilhaft über einen Riemen (54) an den Antriebsmotor (57) der Betätigungseinrichtung (100) angeschlossen ist,

15. Gerät nach Patentanspruch 2, **dadurch gekennzeichnet, dass** die Steuervorrichtung (80) einen Mikroprozessor (82), eine Regeleinheit (83), einen Timer (84) und einen EEPROM (85) sowie Mittel zur manuellen Betätigung dieser Steuervorrichtung (80) umfasst, dass Fühler (89,90) an Eingänge der Steuervorrichtung (80) angeschlossen sind und dass der Motor (57) an einen der Ausgänge der Steuervorrichtung (80) angeschlossen ist.

16. Gerät nach Anspruch 3, **dadurch gekennzeichnet, dass** eine thermoelektrische Vorrichtung (44) im Bereich der ersten Aufnahmestation (41) angeordnet ist und dass diese Vorrichtung (44) eine Peltier-Vorrichtung sein kann.

17. Gerät nach Anspruch 1, **dadurch gekennzeichnet, dass** ein Stativ vorgesehen ist, auf dem die übrigen Teile dieses Gerätes angebracht sind, und dass dieses Stativ so ausgeführt sein kann, dass seine Höhe verstellbar ist.

18. Verfahren zum Betrieb des Gerätes gemäss Patentanspruch 1, **dadurch gekennzeichnet, dass** der Kulturboden (4) während einer vorgegebenen Zeitspanne der Luft derart ausgesetzt wird, dass sich die in der Luft vorhandenen Mikroorganismen auf dem Nährboden (4) ablagern können, dass der Nährboden nach Ablauf einer vorgegebenen Zeitspanne abgedeckt wird, dass nach Ablauf einer Inkubationszeit sichtbare und durch die Keime verursachte Kolonien auf dem Nährboden gezählt werden, woraus sich ein Mass für die Luftkeimzahl ergibt und dass die Bewertung des untersuchten Raumes auf Grund dieser Luftkeimzahl durch "SED"-Einheiten zum Ausdruck gebracht wird.

## Claims

1. Apparatus for the determining the number of microorganisms in air, with an appliance (1) for the settlement of the microorganisms, whereby this appliance (1) shows a lower part (3) and a cover (5), with a nutrient or culture ground (4) which is in the inside of the settlement appliance (1), and with an equipment (10) for the actuation of the cover (5) of the settlement appliance (1), **characterized in that** the appliance shows a first station (41) for the reception of the lower part (3) of the settlement-appliance (1), that the appliance shows a second station (42) for the reception of the cover (5) of the settlement appliance (1), that these reception stations (41,42) are placed side by side and practically on the same level, that the actuation equipment (10) shows an appliance (20) for the handling of the cover (5), that this handling appliance (20) shows a shaft (21), which goes through the mentioned plane, that this shaft (21) is placed between the reception stations (41,42) and that the actuation equipment (10) is executed so, that the cover (5) can be moved between the two reception-stations (41,42) in controlled manner.

2. Apparatus according to patent-claim 1, **characterized in that** the actuation equipment (10) furthermore includes an appliance (80) for the control of the movements of the cover (5).

3. Apparatus according to patent-claim 1, **characterized in that** the handling-appliance (20) comprises bearing means (25) for the cover (5), that the bearing means (25) are assigned to a first end portion of the shaft (21) of the handling-appliance (20) and that means for mechanical movement of the shaft (21) are assigned to the other or second end portion of this shaft (21).

4. Apparatus according to patent-claim 3, **characterized in that** the bearing means (25) include an arm (26), that one end of this arm (26) is connected to the first end portion of the shaft (21) and that a bearer (30) for the cover (5) of the settlement appliance (1) is arranged at the other or free end of the arm (26).

5. Apparatus according to patent-claim3, **characterized in that** the movement means include a motor (57) controllable by a control appliance (80), that the movement means furthermore include a control disk (55), which is driven by the motor (57), that this disk (55) is equipped with control curves (71,72) and that the movement means include also a lever (60) as well as a toothed rack (65) which are coupled on the one hand with the control curves (71,72) on the control disk (55) and with the shaft (21) on the other hand

6. Apparatus according to patent-claim 4, **characterized in that** the arm of the bearing means (25) is pivoted by aid of a tap (27) at the upper end of the shaft (21) and that stops are arranged on the shaft (21) restricting the swing movements of the arm (26), so, that the arm (26) can swing between a practically horizontal and a practically vertical position.

7. Apparatus according to patent-claim 4, **characterized in that** the bearer (30) for the cover (5) of the Petri dish (1) is arranged at the underside of the free end portion of the swing lever (26), that this bearer (30) shows a horizontally extending base plate (31), which has a practically half-circular contour, that the linear extending edge (32) of the base plate (31) of the front side (28) of the swing arm (26) is assigned so, that the edge (32) extends parallel to the front side (28) of the arm (26), that section (33) of the base plate (31) which has practically the shape of a half of a circle, sticks out from the back side (29) of the swing-arm (26), that one essentially vertically extending and also half-circular extending side wall (35) of this bearer (30) hangs down of the half-circular edge (34) of the base plate (31), that a gripping-wall (36) is attached to the lower edge of the side wall (35), that this gripping-wall (36) has the form of a section of a flat and narrow ring, that this gripping wall (36) is attached over its outer edge to the lower edge of the said side wall (35), that the radius of the inside edge (37) on the gripping-ring (36) corresponds to the greatest radius of the cover (5) of the Petri dish (1), i.e. to the radius of the lower edge (7) of the hanging side wall (6) of the cover (5), and that the height of the side wall (35) of the bearer (30) corresponds to the height of the side-wall (6) of the cover (5).

8. Apparatus according to patent-claim 3, **characterized in that** the actuation equipment (10) includes a casing (11), which shows a horizontally extending upper wall (12), that the shaft(21) is pivoted in a bearing (22), which is put into the upper wall (12) of the casing (11), that the bearing (22) is executed so that the shaft (21) in bearing (22) can not only swing but also move in its axial direction to and from, and that the bearing means (25) for the cover (5) of the Petri dish (1) are pivoted at the upper or at that one portion of the shaft (21) which is situated above the upper wall (12) of the casing (11).

9. Apparatus according to patent-claim 8, **characterized in that** a supportplate (50) is arranged in the inside of the casing (11) and below the upper wall (12) of the casing (11), that this plate (50) can be secured by aid of distance-pieces (51) to the underside of the upper wall (12) of the casing (11), that this plate(50) extend practically parallel to the upper wall (12) and bears among other things a second bearing (52) for the lower end portion of the shaft (21), that this second bearing (52) is axial aligned with the first bearing (22), that the low end portion of the shaft (21) is so long, that it protrudes from the second or lower bearing (52), that a control disk (55) is pivoted on the upper side of the support plate (50), that the peripheral part of this disk (55) is equipped with teeth (56), that a driving motor (57) is assigned to this peripheral part of the control disk (55), the pinion (58) of which mesh with the teeth (56) at the control disk (55)

10. Apparatus according to patent-claim 9, **characterized in that** at least one bearing block (59) sticks out from underside of the support-plate (50), in which a two-armed angle lever (60) is pivoted in a swinging manner, that the angle between the arms (61,62) the angle-lever (60) amounts to approximately 90 degrees, that the free end of the first arm (61) of the angle lever (60) is secured with help of a tap (68) at the lower end of the shaft (21), that the second arm (62) of the angle lever (60) goes through a corresponding opening in the support plate (50), so, that the free end portion of the second arm (62) of the angle lever (60) lies in the area of the control disk (55), in fact beneath of this, that the free end of the second arm (62) of the angle-lever (60) is equipped with a role (63) and that the surface of this role (63) has the form of the surface of a ball layer, whereby the equator of the ball is in the middle of the height of this ball layer.

11. Apparatus according to patent-claim 3, **characterized in that** the shaft (21) is provided with a toothed rim (64) **in that** one region thereof which extend between the bearings (22,52), that the toothed rim (64) surrounds the shaft (21) and is secured to this shaft (21), that a toothed rack (65) is provided for which extends horizontally and which is movable, that this toothed rack (65) is placed over the control disk (55) and that the tooth's thereof mesh with toothed rim (64) on the shaft (21) and that underside of the toothed rack (65) carries a leading role (67) which sticks out practically rectangularly from the main body of the tooth-rack (65).

12. Apparatus according to patent-claim 10, **characterized in that** in the upper and in the lower extensive side of the control disk (55) each a cam (71,72) is executed, that these cams (71,72) can be carried out as deepenings or grooves, which have an essentially rectangular cross-section, that the first cam (71) has essentially the form of a spiral with only one single loop, that the role (63) of the second arm (62) lies in the lower cam (71), that the second cam (72) is non circular and shows sections, which make possible a horizontal movement of the bearer (30) of the cover (5) going back and forth or a halt of this bearer (30) and that the leading role (67) of the tooth-rack (65) lies in the upper cam (72).

13. Apparatus according to patent-claim 1, **characterized in that** the actuation equipment (100) includes a coulisse (101), which is essentially cup- or cartridge-shaped, that this coulisse (101) shows a ground (102) and a cylindrical side-wall (103) extending away from the ground (102), that a helicoidal shaped groove (104) is carried out in this side-wall (103), that the swing shaft (21) of the handling appliance (20) goes through an opening (99) in the ground (102) of the coulisse (101), that it is arranged in this opening (99) movable in a swinging manner and also alongside, that the actuation-equipment (100) furthermore includes a leading plate (105), that this plate (105) shows a vertically extending leading edge (107) and slit (06), that this slit (60) adjoins to the upper end of the leading edge (107) and extends practically horizontally, that the actuation equipment (100) also includes a stop (111) which is essentially platen shaped, whereby a slit exists between the leading edge (107) of the leading plate (105) and that one vertical edge (118) of the stop plate (111) which is next lying to the leading plate (105), that one end of a bolt (120) is secured to the shaft (21), that this bolt sticks out from this shaft (21), that this bolt (120) is arranged on the shaft (21) so, that it can go through the groove (104) in the coulisse (101) and that the bolt (120) is executed so long, that his/its free End portion lies in the slit.

14. Apparatus according to patent-claim 13, **characterized in that** a hollow shaft (96) is in the middle of the ground (102) of the coulisse, whereby the opening in this shaft (96) matches the opening (99) in the ground (102) of the coulisse, that the bearing shaft (96) is arranged in a bearing (97) in a rotating or swinging manner, that this bearing (97) is put into a bearing plate (95) of the appliance casing, so that a part of the bearing shaft (96) sticks out from the underside of the bearing (97), that this free end of the bearing shaft (96) is equipped with a disk (98), which is connected advantageously over a belt (54) to the driving motor (57) of the actuation-equipment (100),

15. Apparatus according to patent-claim 2, **characterized in that** the control appliance (80) includes a microprocessor (82), a control unit (83), a timer (84) and an EEPROM (85) as well as means for the manual actuation of this control-appliance (80), that detecting elements (89,90) are connected to the inputs of the control appliance (80) and that the motor (57) is connected to one of the outputs of the control appliance (80).

16. Apparatus according to claim 3, **characterized in that** a thermoelectric appliance (44) is arranged in the area of the first reception station (41) and that this appliance (44) can be a Peltier- appliance.

17. Apparatus according to claim 1, **characterized in that** a tripod is provided for, on which the remaining parts of this apparatus are arranged, and that this tripod can be executed so, that its height is adjustable.

18. Method for operating the apparatus according to patent-claim 1, **characterized in that** the culture ground (4) is exposed to the air during a predetermined span so, that the microorganisms existing in the air can deposit themselves on the culture ground (4), that the culture ground is covered after course of a predetermined span, that after a course of an incubation period visible and through the germs caused colonies on the culture ground are counted, from which a measurement results for the air-germ-number and that the evaluation of the examined area is brought for the expression on the basis of this air-germ-number by "SED"-units.

## Revendications

1. Appareil pour la détermination du nombre de micro-organismes dans l'air, avec un dispositif (1) pour déposer les micro-organismes, ce dispositif (1) présentant une partie inférieure (3) et un couvercle (5) muni d'un fond nutritif ou de culture (4) qui se trouve à l'intérieur du dispositif de dépôt (1) et muni d'un mécanisme (10) pour actionner le couvercle (5) du dispositif de dépôt (1), **caractérisé en ce que** l'appareil présente une première station (41) qui est conçue pour recevoir la partie inférieure (3) du dispositif de dépôt (1), que l'appareil présente une deuxième station (42) qui est conçue pour recevoir le couvercle (5) du dispositif de dépôt (1), que ces stations de réception (41, 42) se trouvent l'une à côté de l'autre et pratiquement sur le même plan, que le mécanisme d'actionnement (10) présente un dispositif (20) pour manipuler le couvercle (5), que ce dispositif de manipulation (20) présente un axe (21) qui passe à travers ledit plan, que cet axe (21) est disposé entre les stations de réception (41, 42) et que le mécanisme d'actionnement (10) est réalisé de telle manière que le couvercle (5) puisse se déplacer de manière commandée entre les deux stations de réception (41, 42).

2. Appareil selon la revendication 1, **caractérisé en ce que** le mécanisme d'actionnement (10) comprend en plus un dispositif (80) pour commander les mouvements du couvercle (5).

3. Appareil selon la revendication 1, **caractérisé en ce que** le dispositif de manipulation (20) présente de moyens supports (25) pour le couvercle (5), que les moyens supports (25) sont associés à une première partie finale de l'axe (21) du dispositif de manipulation (20) et que des moyens de déplacement mécanique de l'axe (21) sont associés à l'autre ou à la deuxième partie finale de cet axe (21).

4. Appareil selon la revendication 3, **caractérisé en ce que** les moyens porteurs (25) comprennent un bras (26), que ce bras (26) est relié par l'une de ses extrémités à la première partie finale de l'axe (21) et qu'un support (30) pour le couvercle (5) du dispositif de dépôt (1) est monté sur l'autre extrémité libre du bras (26).

5. Appareil selon la revendication 3, **caractérisé en ce que** les moyens de déplacement comprennent un moteur (57) qui peut être commandé par un dispositif de commande (80), que les moyens de commande comprennent en outre un disque de commande (55) qui peut être entraîné par le moteur (57), que ce disque (55) est muni de cames de commande (71, 72) et que les moyens de déplacement comprennent également un levier (60) ainsi qu'une crémaillère (65) qui sont reliés d'un côté avec les cames de commande (71, 72) sur le disque de commande (55) et de l'autre côté avec l'axe (21).

6. Appareil selon la revendication 4, **caractérisé en ce que** le bras des moyens supports (25) est fixé de manière articulée à l'extrémité supérieure de l'axe (21) à l'aide d'un tenon (27) et que des butées sont façonnées sur l'axe (21), lesquelles limitent les mouvements de basculement du bras (26) de manière à ce que le bras (26) puisse basculer entre une position pratiquement horizontale et une position pratiquement verticale.

7. Appareil selon la revendication 4, **caractérisé en ce que** le support (30) pour le couvercle (5) de la boîte de Pétri (1) est monté sur le dessous de la partie finale libre du levier basculant (26), que ce support (30) présente une plaque de base (31) horizontale qui a un contour pratiquement semi-circulaire, que le bord rectiligne (32) de la plaque de base (31) est affectée au côté avant (28) du bras basculant (26) de manière à ce que ce bord (32) soit parallèle à la face avant (28) du bras (26) et que la section pratiquement semi-circulaire (33) de la plaque de base (31) soit éloignée de la face arrière (29) du bras basculant (26) de manière à ce qu'une paroi latérale (35) essentiellement verticale et également semi-circulaire de ce support (30) pende vers le bas depuis le bord semi-circulaire (34) de la plaque de base (31), qu'une paroi de préhension (36) se rattache au bord inférieur de la paroi latérale (35), laquelle a la forme d'une section d'un anneau plat et étroit, que le bord extérieur de cette paroi de préhension (36) se rattache à l'arête du bord inférieur de ladite paroi latérale (35), que le rayon du bord intérieur (37) sur l'anneau de préhension (36) correspond au rayon de la partie inférieure (3) de la boîte de Pétri (1), que le rayon de la paroi latérale (35) du support (30), par contre, correspond au rayon le plus grand du couvercle (5) de la boîte de Pétri (1), c'est à dire au rayon du bord inférieur (7) de la paroi latérale (6) pendue vers le bas du couvercle (5) et que la hauteur de la paroi latérale (35) du support (30) correspond à la hauteur de ladite paroi latérale (6) du couvercle (5).

8. Appareil selon la revendication 3, **caractérisé en ce que** le mécanisme d'actionnement (10) comprend un boîtier (11) qui présente une paroi supérieure horizontale (12), que l'axe (21) est logé dans un palier (22) qui est inséré dans la paroi supérieure (12) du boîtier (11), que le palier (22) est réalisé de telle manière que l'axe (21) puisse non seulement basculer dans le palier (22), mais également y effectuer un mouvement de va-et-vient, que les moyens supports (25) pour le couvercle (5) de la boîte de Pétri (1) sont fixés de manière articulée à la partie finale de l'axe (21) supérieure ou se trouvant au-dessus de la paroi supérieure (12) du boîtier (11)

9. Appareil selon la revendication 8, **caractérisé en ce qu'**une plaque auxiliaire (50) est disposée à l'intérieur du boîtier (11) et sous la paroi supérieure (12) du boîtier (11), laquelle peut être fixée sur le dessous de la paroi supérieure (12) du boîtier (11) à l'aide de pièces d'écartement (51), que cette plaque auxiliaire (50) est pratiquement parallèle à la paroi supérieure (12) et elle porte entre autres un deuxième palier (52) pour la partie finale inférieure de l'axe (21), que ce deuxième palier (52) est aligné dans le sens axial avec le premier palier (22), que la partie finale inférieure de l'axe (21) est réalisée suffisamment longue pour qu'elle dépasse du deuxième palier, inférieur (52), qu'un disque de commande (55) est logé de manière à pouvoir tourner sur le dessus de la plaque auxiliaire (50) dont le pourtour est muni de dents (56), que ce pourtour du disque de commande (55) est associé à un moteur d'entraînement (57) dont le pignon (58) vient s'engager avec les dents (56) sur le disque de commande (55).

10. Appareil selon la revendication 9, **caractérisé en ce qu'**au moins un palier support (59) s'écarte du dessous de la plaque auxiliaire (50), sur lequel un levier coudé (60) à deux bras est fixé de manière à pouvoir pivoter, que l'angle entre les bras (61, 62) de ce levier coudé (60) est d'environ 90 degrés, que l'extrémité libre du premier bras (61) du levier coudé (60) est fixé de manière articulée à l'extrémité inférieure de l'axe (21) à l'aide d'un tenon (58), que le deuxième bras (62) du levier coudé (60) passe à travers une ouverture correspondante dans la plaque auxiliaire (50) de manière à ce que la partie finale du deuxième bras (62) du levier coudé (60) se trouve dans la zone du disque de commande (55), à savoir sous celui-ci, que l'extrémité libre du deuxième bras (62) du levier coudé (60) est munie d'un galet (63), que la surface de ce galet (63) a la forme de la surface d'un segment sphérique avec lequel l'équateur de la sphère se trouve au centre de la hauteur de ce segment sphérique.

11. Appareil selon la revendication 3, **caractérisé en ce que** dans chaque zone de l'axe (21) qui se trouve entre les deux paliers (22) et (52), l'axe (21) est muni d'une couronne dentée (64) qui entoure l'axe (21) et qui est reliée à demeure avec l'axe (21), qu'il est prévu une crémaillère (65) disposée horizontalement et coulissante qui se trouve au-dessus du disque de commande (55) et dont les dents (66) viennent s'engager avec la couronne dentée (64) sur l'axe (21), que le côté inférieur de la crémaillère (65) comporte un galet de guidage (67) qui s'écarte est pratiquement perpendiculairement au corps de base de la crémaillère (65).

12. Appareil selon la revendication 10, **caractérisé en ce qu'**une came de commande (71, 72) est à chaque fois réalisée dans le côté supérieur et dans le côté inférieur à grande surface du disque de commande (55), que ces cames de commande (71, 72) peuvent être réalisées sous la forme de creux ou de nervures qui présentent une section essentiellement rectangulaire, que la première came de commande (71) a pour l'essentiel la forme d'une spirale avec un seul enroulement, que le galet (63) du deuxième bras (62) du levier coudé (60) se trouve dans la came de commande inférieure (71), que la deuxième came de commande (72) et excentrique et présente des sections qui permettent un mouvement de va-et-vient horizontal du support (30) du couvercle (5) ou une immobilisation de ce support (30), que le galet de guidage (67) repose sur la crémaillère (65) dans la came de commande supérieure (72).

13. Appareil selon la revendication 1, **caractérisé en ce que** le mécanisme d'actionnement (100) comprend une coulisse (101) qui a pour l'essentiel la forme d'une coupe ou d'une douille, que cette coulisse (101) présente un fond (102) et une paroi latérale cylindrique (103) qui s'en éloigne, qu'une rainure (104) en forme de pas de vis est réalisée dans cette paroi latérale (103), que l'axe de pivotement (21) du dispositif de manipulation (20) passe à travers une ouverture (99) dans le fond (102) de la coulisse (101) et il est logé dans cette ouverture (99) à la fois de manière à pouvoir pivoter et de manière à pouvoir coulisser dans le sens longitudinal, que le mécanisme d'actionnement (100) comprend également une plaque de guidage (105), que cette plaque de guidage (105) présente un bord de guidage (107) qui s'étend dans le sens vertical et une fente (06) pratiquement horizontale qui vient se raccorder à l'extrémité supérieure du bord de guidage (107), que le mécanisme d'actionnement (100) comprend également une butée (111) ayant essentiellement la forme d'une plaque, un écart existant ici entre le bord de guidage (107) de la plaque de guidage (105) et la plaque de guidage (105) au niveau du bord vertical (118) le plus proche de la plaque de butée (111), qu'un boulon (120) est fixé d'un côté sur l'axe (21) et s'écarte de cet axe (21), que ce boulon (120) est disposé sur l'axe (21) de manière à ce qu'il puisse passer à travers la rainure (104) dans la coulisse (101) et que le boulon (120) est suffisamment long pour que sa partie finale libre repose dans ledit écart.

14. Appareil selon la revendication 13, **caractérisé en ce qu'**un axe creux (96) se trouve au centre du fond de la coulisse (102), l'ouverture dans cet axe (96) correspondant ici à l'ouverture dans le fond de la coulisse (102), que l'axe support (96) est logé dans un palier (97) de manière à pouvoir tourner ou pivoter, que ce palier (97) se trouve dans une plaque support (95) du boîtier de l'appareil de manière à ce qu'une partie de l'axe support (96) s'écarte du dessous du palier (97), que cette extrémité libre de l'axe support (96) est munie d'une poulie (98) qui est avantageusement rattachée par le biais d'une courroie (54) au moteur d'entraînement (57) du mécanisme d'actionnement (100).

15. Appareil selon la revendication 2, **caractérisé en ce que** le dispositif de commande (80) comprend un microprocesseur (82), une unité de régulation (83), un temporisateur (84) et une EEPROM (85) ainsi que des moyens pour actionner manuellement ce dispositif de commande (80), que des capteurs (89, 90) sont raccordés aux entrées du dispositif de commande (80) et que le moteur (57) est raccordé à l'une des sorties du dispositif de commande (80).

16. Appareil selon la revendication 3, **caractérisé en ce qu'**un dispositif thermoélectrique (44) est disposé dans la zone de la première station de réception (41) et que ce dispositif (44) peut être un dispositif de Peltier.

17. Appareil selon la revendication 1, **caractérisé en ce qu'**il est prévu un pied sur lequel sont montées les autres parties de cet appareil et que ce pied peut être réalisé de manière à pouvoir être réglable en hauteur.

18. Procédé pour utiliser l'appareil selon la revendication 1, **caractérisé en ce que** le fond de culture (4) est exposé à l'air pendant une période préfinie, que les micro-organismes présents dans l'air peuvent de déposer sur le fond nutritif (4), que le fond nutritif (4) est couvert après écoulement d'une période prédéfinie, que les colonies visibles et provoquées par les germes sont comptées sur le fond nutritif après une période prédéfinie, ce qui donne une mesure du nombre de germes dans l'air et que l'évaluation de l'espace analysé est imprimée sur la base de ce nombre de germes dans l'air par « unités SED ».
